(19) Europäisches Patentamt
European Patent Office
Office européen des brevets

(11) Numéro de publication: **0 046 606**
**A1**

(12) # DEMANDE DE BREVET EUROPEEN

(21) Numéro de dépôt: 81200777.1

(22) Date de dépôt: 07.07.81

(51) Int. Cl.³: C 07 D 303/48
C 07 D 303/32
//C07C69/73, C07C49/573,
C07C69/52, C07C69/608,
C07C49/543, C07C61/22

(30) Priorité: 26.08.80 CH 6413/80

(43) Date de publication de la demande:
03.03.82 Bulletin 82/9

(84) Etats contractants désignés:
CH DE FR GB LI NL

(71) Demandeur: FIRMENICH SA
Case postale 239
CH-1211 Genève 8(CH)

(72) Inventeur: Schulte-Elte, Karl-Heinrich
44, Chemin Carabot
CH-1213 Onex/Ge(CH)

(72) Inventeur: Egger, Bernard
6, chemin Guillon
CH-1233 Bernex/Ge(CH)

(72) Inventeur: Müller, Bernard
16, route de Malagnou
CH-1208 Geneve(CH)

(74) Mandataire: Salvadori, Giuseppe, Dr.
c/o Firmenich S.A., Case Postale 239
CH-1211 Genève 8(CH)

(54) **Composés alicycliques oxygénés, procédé pour leur préparation et leur utilisation à titre de produits de départ pour la préparation de composés cyclohexadiéniques.**

(57) Les composés alicycliques oxygénés nouveaux de formule

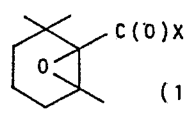

(I)

(X = OH, alkyle, O-alkyle) sont utilisés à titre de produits de départ pour la préparation des composés de formule

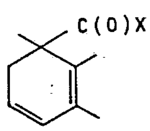

(III)

(X = OH, alkyle, O-alkyle). Ladite utilisation consiste en un traitement au moyen d'un agent déshydratant acide.

Procédé pour la préparation desdits composés de formule (I).

EP 0 046 606 A1

Croydon Printing Company Ltd.

Composés alicycliques oxygénés, procédé pour leur préparation et leur utilisation à titre de produits de départ pour la préparation de composés cyclohexadiéniques

L'invention a pour objet des composés alicycliques oxygénés nouveaux, plus précisément de formule

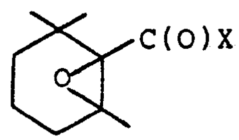

(I)

(X = OH, alkyle, O-alkyle), utiles à titre de produits de départ pour la préparation des composés cyclohexadiéniques correspondants, de formule

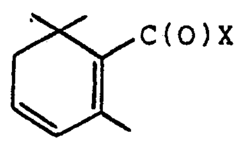

(III)

(X = OH, alkyle, O-alkyle).

Elle a également pour objet l'utilisation desdits composés de formule (I) mentionnée ci-dessus, ainsi qu'un procédé pour leur préparation. Elle est définie aux revendications.

Dans la définition du symbole X donnée ci-dessus, le terme alkyle sert à désigner un radical alkyle inférieur, contenant de préférence de 1 à 3 atomes de carbone, méthyle, éthyle ou propyle par exemple.

Diverses publications décrivent la préparation des composés cyclohexadiéniques définis ci-dessus : dans le cas

de la 2,6,6-triméthyl-cyclohexa-1,3-diène-1-yl méthyle cétone ($X = CH_3$ dans la formule III) par exemple, on peut citer Chem. Comm. 1973, 161. L'acide 2,6,6-triméthyl-cyclohexa-1,3-diène-1-carboxylique, de même que les esters méthylique et éthylique correspondants ($X = OH$, respectivement $OCH_3$ et $OC_2H_5$ dans la formule III), s'obtiennent à partir du composé mono-insaturé correspondant, de formule

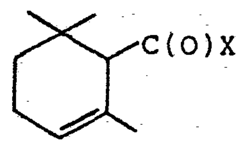

(IV)

($X = OH$, $OCH_3$, $OC_2H_5$), après addition d'halogène et élimination subséquente d'hydracide halogéné, comme indiqué, par exemple, dans Compt. Rend. Sér. C, 262, 1725 (1966) et Helv. Chim. Acta 38, 1863 (1955).

Lors de leur application à l'échelle industrielle cependant, de telles méthodes se révèlent être d'un emploi malaisé et peu rentables. Par l'utilisation des composés alicycliques oxygénés de formule (I) telle que définie au sein de la présente invention, on parvient à préparer lesdits composés de formule (III) dans des conditions nettement plus favorables, tant du point de vue pratique qu'économique.

Comme indiqué précédemment, les composés de formule (I) sont des composés nouveaux et l'on peut notamment citer à ce propos l'acide 2,6,6-triméthyl-1,2-époxy-cyclohexane-1-carboxylique, le 2,6,6-triméthyl-1,2-époxy-cyclohexane-1-carboxylate de méthyle, le 2,6,6-triméthyl-1,2-époxy-cyclohexane-1-carboxylate d'éthyle et la 2,6,6-triméthyl-1,2-époxy-cyclohexyl méthyl cétone.

Lesdits composés s'obtiennent suivant l'invention à partir des composés de formule

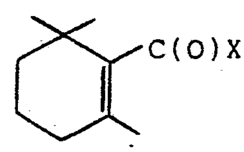

(II)

dans laquelle le symbole X est défini comme indiqué précédemment, après traitement au moyen d'un peracide organique,

plus particulièrement un peracide organique à pouvoir époxydant élevé tel, par exemple, l'acide permaléique, m-chloroperbenzoïque, performique, perphtalique ou mono-chloroperacétique. Pour des raisons d'ordre pratique et économique, on utilise de préférence l'acide permaléique.

Ledit traitement s'effectue selon les conditions usuelles en chimie organique, en présence d'un solvant organique inerte, tel un hydrocarbure, le toluène par exemple, ou un hydrocarbure chloré, le chlorure de méthylène ou le trichloro-éthane par exemple. On opère en outre à une température généralement comprise entre environ 0 et 50°C, plus particulièrement comprise entre environ 30 et 40°C.

Les composés monoinsaturés de formule

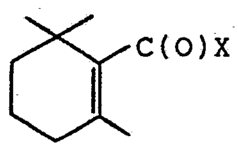

(II)

(X = OH, alkyle, O-alkyle), utilisés comme produits de départ dans le procédé de l'invention, sont des produits connus que l'on peut aisément obtenir conformément aux procédés décrits dans la littérature scientifique. On peut citer par exemple, dans les cas suivants :

X = OH : Compt. Rend. Sér. C, 262, 1725 (1966)

$X = OCH_3$ : Chem. Abstr. 57, 1240 g (1962)

$X = CH_3$ : Chem. Comm. 1973, 161.

Conformément à l'objet de la présente invention, les composés de formule (I) définis plus haut peuvent être soumis à l'action d'un agent déshydratant acide, pour être ainsi convertis en composés cyclohexadiéniques de formule (III) correspondants.

A titre d'agent déshydratant acide, on utilise avantageusement un acide fort, minéral ou organique tel l'acide phosphorique, perchlorique, sulfurique, p-toluènesulfonique, voire l'hydrogénosulfate de potassium par exemple. Pour des raisons d'ordre pratique et économique, on utilise de préférence une terre de diatomées acide, en présence d'un solvant organique inerte tel un éther, le dioxanne, ou une cétone, la diéthyl cétone par exemple. Le

traitement du composé époxydé de formule (I) au moyen de l'agent déshydratant acide sus-mentionné s'effectue le plus généralement à une température comprise entre environ 90 et 110°C, de préférence au voisinage de l'ébullition du solvant ou mélange de solvants choisi.

Au cours d'un tel traitement, il se forme un composé hydroxylé intermédiaire nouveau, de formule

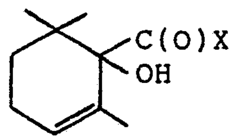

(V)

(X = OH, alkyle, O-alkyle), que l'on peut isoler et identifier selon les conditions réactionnelles appliquées. Cette opération n'est cependant pas nécessaire au bon déroulement du traitement décrit plus haut, la réaction étant poursuivie jusqu'à l'obtention du composé de formule (III) désiré.

L'invention sera illustrée de façon plus détaillée à l'aide des exemples ci-après (températures en degrés centigrades).

## Exemple 1

2,6,6-Triméthyl-1,2-époxy-cyclohexane-1-carboxylate de méthyle

A 91,0 g (0,50 mole) de β-cyclogéraniate de méthyle, 73,5 g (0,75 mole) d'anhydride maléique en mélange avec 250 ml de $CH_2Cl_2$, on a ajouté, sous agitation, 25,0 g (0,75 mole) de peroxyde d'hydrogène à 70%. Cette addition a été effectuée goutte à goutte, de façon à maintenir la température du mélange de réaction entre 30 et 38°. Après avoir poursuivi l'agitation du mélange jusqu'à disparition du β-cyclogéraniate de départ (au total 5 h), refroidi ledit mélange et filtré l'acide maléique formé, on a successivement lavé la phase organique avec $H_2O$, $Na_2CO_3$ saturé puis $H_2O$ jusqu'à neutralité. Après séchage sur $Na_2SO_4$, concentration et distillation, on a recueilli 91,1 g (rendement 92%) du produit désiré, Eb. 45°/13,3 Pa.

IR : 1740 $cm^{-1}$;

RMN : 1,08 (3H, s), 1,14 (3H, s); 1,26 (3H, s); 3,77 (3H,

s) δ ppm;

SM : $M^+ = 198$ (2); m/e = 183 (7), 166 (8), 155 (12), 142 (53), 123 (22), 115 (49), 95 (51), 85 (35), 69 (74), 55 (64), 43 (100), 41 (65), 29 (21).

## Exemple 2

**2,6,6-Triméthyl-1,2-époxy-cyclohexane-1-carboxylate d'éthyle**

98,0 g (0,50 mole) de β-cyclogéraniate d'éthyle ont été traités au moyen d'anhydride maléique et de peroxyde d'hydrogène comme indiqué à l'Exemple 1, pour donner le produit désiré avec un rendement de 90%.

Eb. $55^o$/13,3 Pa

IR : 1735 $cm^{-1}$;

RMN : 1,06 (3H, s); 1,13 (3H, s); 1,23 (3H, s); 4,22 (2H, 2t) δ ppm;

SM : $M^+ = 212$ (5); m/e = 197 (3), 184 (3), 166 (9), 156 (48), 139 (24), 129 (48), 111 (34), 95 (51), 87 (40), 69 (83), 55 (65), 43 (100), 41 (60), 29 (55).

## Exemple 3

**2,6,6-Triméthyl-1,2-époxy-cyclohexyl méthyl cétone**

8,3 g (0,05 mole) de 2,6,6-triméthyl-cyclohex-1-ényl méthyl cétone ont été traités au moyen d'anhydride maléique et de peroxyde d'hydrogène selon le procédé de l'Exemple 1 pour donner, après les traitements d'extraction et de purification décrits plus haut, 8,5 g (rendement 95%) du produit désiré, Eb. $85^o/7{,}98 \times 10^2$ Pa.

IR : 1700 $cm^{-1}$;

RMN : 1,07 (3H, s); 1,13 (3H, s); 1,17 (3H, s); 2,20 (3H, s) δ ppm;

SM : $M^+ = 182$ (0,5); m/e = 140 (12), 125 (45), 111 (35), 96 (14), 84 (10), 69 (70), 55 (71), 43 (100), 27 (10).

## Exemple 4

**Acide 2,6,6-triméthyl-1,2-époxy-cyclohexane-1-carboxylique**

84 g (0,50 mole) d'acide β-cyclogéranique ont été traités au moyen de 73,5 g (0,75 mole) d'anhydride maléique et 25,0 g (0,75 mole) de peroxyde d'hydrogène à 70% aux

conditions décrites à l'Exemple 1. Après refroidissement, le mélange réactionnel a été extrait avec NaOH à 10% dans $H_2O$ et les extrais aqueux neutralisés à l'aide de $H_2SO_4$ à 10% dans $H_2O$ pour donner, après évaporation, 55,3 g (rendement 60%) du produit désiré (pureté 75% selon analyse par chromatographie sur couche mince). Un échantillon pour analyse a été identifié comme suit :

IR : 3300, 1750 $cm^{-1}$;

RMN : 1,05 (3H, s); 1,26 (3H, s); 1,32 (3H, s) $\delta$ ppm;

SM : $M^+ = 184$ (1).

Exemple 5

2,6,6-Triméthyl-1,2-époxy-cyclohexane-1-carboxylate de méthyle

A 18,2 g (0,10 mole) de β-cyclogéraniate de méthyle en solution dans 125 ml de $CH_2Cl_2$, on a ajouté par petites portions 24,6 g (0,11 mole) d'acide m-chloroperbenzoïque (température : $0^o$). Après addition, l'agitation du mélange a été poursuivie durant 3 h puis le mélange de réaction a été entreposé durant 24 h à température ambiante. Après filtration, lavage avec NaOH 2N, puis avec $H_2O$ jusqu'à neutralité, séchage sur $Na_2SO_4$, concentration et distillation du résidu sous pression réduite (13,3 Pa), on a isolé 19,6 g (rendement 84%) du produit désiré.

Exemple 6

Préparation de 2,6,6-triméthyl-cyclohexa-1,3-diène-1-carboxylate de méthyle

99,0 g (0,50 mole) de 2,6,6-triméthyl-1,2-époxy-cyclohexane-1-carboxylate de méthyle ont été ajoutés à 50 g de terre de diatomées acide (préalablement séchée par distillation azéotropique avec du toluène) en suspension dans 200 ml de dioxanne. Après chauffage du mélange durant 4 h à reflux ($100^o$), sous atmosphère d'azote et avec une bonne agitation, on a filtré le mélange refroidi sur CELITE et concentré la phase organique. Après distillation du résidu, on a finalement recueilli 64,4 g (rendement 65%) du produit désiré, Eb. $52^o/9,31 \times 10^2$ Pa.

Le produit ainsi obtenu est identique à un échantillon préparé selon Compt. Rend. Sér. C, 262, 1725 (1966).

En cours de réaction, on a isolé et identifié le composé de formule

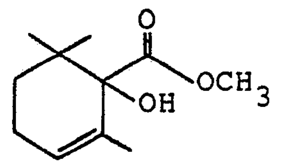

IR : 3500, 1730 $cm^{-1}$;

RMN : 0,83 (3H, s); 0,99 (3H, s); 1,59 (3H, m); 3,88 (3H, s); 5,66 (1H, m) δ ppm;

SM : $M^+$ = 198 (2); m/e = 166 (5), 138 (33), 130 (13), 121 (7), 111 (20), 95 (47), 82 (21), 70 (49), 69 (100), 55 (32), 43 (68), 27 (11).

## Exemple 7

### Préparation de 2,6,6-triméthyl-cyclohexa-1,3-diène-1-carboxylate d'éthyle

105 g (0,50 mole) de 2,6,6-triméthyl-1,2-époxy-cyclohexane-1-carboxylate d'éthyle ont été traités au moyen de 50 g de terre de diatomées acide comme indiqué à l'Exemple 6 (4 h à 100°). On a recueilli le produit désiré avec un rendement de 62%.

Le produit ainsi obtenu est identique à un échantillon préparé selon Helv. Chim. Acta 38, 1863 (1955).

En cours d'analyse, on a isolé et identifié le composé de formule

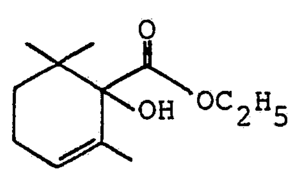

IR : 3500, 1720 $cm^{-1}$;

RMN : 0,83 (3H, s), 0,98 (3H, s); 1,3 (3H, t, J = 3 Hz); 1,6 (3H, m); 2,08 (2H, m); 4,21 et 4,32 (2H, 2t, J = 3 Hz); 5,63 (1H, m) δ ppm;

SM : $M^+$ = 212 (1); m/e = 139 (77), 121 (4), 95 (42), 82 (26), 55 (6), 43 (100), 29 (7).

Exemple 8

Préparation de 2,6,6-triméthyl-cyclohexa-1,3-diène-1-yl méthyl cétone

2,0 g (0,011 mole) de 2,6,6-triméthyl-1,2-époxy-cyclohexyl méthyl cétone ont été traités au moyen de 0,5 g de terre de diatomées acide dans 10 ml de dioxanne à 100° pour donner, après les traitements décrits à l'Exemple 6, 1,3 g (rendement 72%) du produit désiré (pureté 80%).

Un échantillon pour analyse s'est montré, après identification, comparable au produit préparé selon Chem. Comm. 1973, 161.

En cours de réaction, on a également isolé et identifié le composé de formule

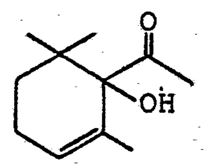

IR : 3450, 1700 $cm^{-1}$;

RMN : 0,8 (3H, s); 1,0 (3H, s); 1,5 (3H, m); 2,22 (3H, s); 4,1 (1H, large s); 5,7 (1H, m) δ ppm;

SM : m/e = 139 (35), 95 (30), 81 (5), 67 (5), 55 (7), 43 (100).

Exemple 9

Préparation d'acide 2,6,6-triméthyl-cyclohexa-1,3-diène-1-carboxylique

12,2 g d'acide 2,6,6-triméthyl-1,2-époxy-cyclohexane-1-carboxylique (pureté environ 75% ; voir Exemple 4) ont été chauffés à 100° en présence de 5 g de terre de diatomées acide et de 50 ml de dioxanne jusqu'à disparition totale du produit de départ (contrôle par chromatographie sur couche mince). Après les traitements d'extraction et de purification décrits à l'Exemple 4, on a recueilli 4,6 g d'un mélange contenant environ 55% du produit désiré selon l'analyse spectroscopique ci-après.

RMN : 1,18 (6H, 2s); 1,96 (3H, s); 2,14 (2H, d, J = 3 Hz); 5,9 (1H, m); 8,7 (1H, large m) δ ppm.

L'acide désiré peut être également isolé et purifié après estérification du mélange réactionnel, séparation par chromatographie en phase gazeuse préparative et saponification.

REVENDICATIONS

1. Composés alicycliques oxygénés de formule

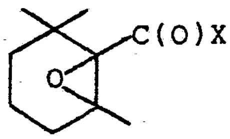

(I)

dans laquelle le symbole X représente un groupe OH ou un radical alkyle ou O-alkyle, le reste alkyle représentant un alkyle inférieur de préférence de $C_1$ à $C_3$.

2. A titre de composés selon la revendication 1, les composés ci-après :

- acide 2,6,6-triméthyl-1,2-époxy-cyclohexane-1-carboxylique,
- 2,6,6-triméthyl-1,2-époxy-cyclohexane-1-carboxylate de méthyle,
- 2,6,6-triméthyl-1,2-époxy-cyclohexane-1-carboxylate d'éthyle,
- 2,6,6-triméthyl-1,2-époxy-cyclohexyl méthyl cétone.

3. Procédé pour la préparation d'un des composés suivant la revendication 1, caractérisé en ce qu'on traite au moyen d'un peracide organique un composé de formule

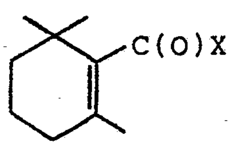

(II)

dans laquelle le symbole X est défini comme indiqué à la revendication 1 pour la formule (I).

4. Procédé selon la revendication 3, caractérisé en ce que le traitement du composé de formule (II) s'effectue au moyen d'acide permaléique, m-chloroperbenzoïque, performique, perphtalique ou mono-chloroperacétique.

5. Utilisation d'un des composés suivant la revendication 1 à titre de produit de départ pour la préparation d'un composé de formule

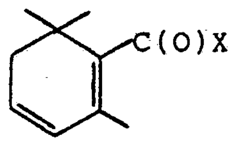

(III)

dans laquelle le symbole X est défini comme indiqué à la revendication 1 pour la formule (I), caractérisée en ce qu'on soumet ledit composé de formule (I) à l'action d'un agent déshydratant acide, en présence d'un solvant organique inerte.

6. Utilisation selon la revendication 5, caractérisée en ce que l'agent déshydratant acide est un acide fort, minéral ou organique et le solvant organique inerte un éther ou une cétone.

7. Utilisation selon la revendication 5, caractérisée en ce que le traitement du composé de formule (I) au moyen de l'agent déshydratant acide s'effectue à une température comprise entre 90 et 110°C.

0046606

Office européen des brevets

**RAPPORT DE RECHERCHE EUROPEENNE**

Numéro de la demande
EP 81 20 0777

**DOCUMENTS CONSIDERES COMME PERTINENTS**

| Catégorie | Citation du document avec indication, en cas de besoin, des parties pertinentes | Revendication concernée | CLASSEMENT DE LA DEMANDE (Int. Cl.³) |
|---|---|---|---|
| X | HELVETICA CHIMICA ACTA, vol. 58 no. 7, 5-11-1975, Bâle, CH<br>E.P. MULLER et al.: "Zur Photochemie der α,β-Epoxy-ketone: γ-Wasserstoffabstraktion versus Epoxy-keton-Umlagerung", pages 2173-2188<br>* Page 2174, formule 6; page 2181 *<br>-- | 1-4 | C 07 D 303/48<br>303/32//<br>C 07 C 69/73<br>49/573<br>62/52<br>69/608<br>49/543<br>61/22 |
| X | HELVETICA CHIMICA ACTA, vol. 54, no. 7, 1-11-1971<br>Bâle, CH<br>K.H. SCHULTE-ELTE et al.:"Die farbstoffsensibilisierte Photo-Oxygenierung von β-Damascol. Ein einfaches Verfahren zur Darstellung von β-Damascenon", pages 1899-1910<br>* En entier *<br>-- | 1-7 | DOMAINES TECHNIQUES RECHERCHES (Int. Cl.³)<br>C 07 D 303/48<br>303/32 |
| X | GB - A - 864 446 (UNION CARBIDE)<br>* Pages 13,14, exemple 18; pages 16,17; revendications *<br>---- | 1-4 | |

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

X Le présent rapport de recherche a été établi pour toutes les revendications

| Lieu de la recherche | Date d'achèvement de la recherche | Examinateur |
|---|---|---|
| La Haye | 19-11-1981 | ALLARD |

OEB Form 1503.1 06.78